# EUROPÄISCHE PATENTSCHRIFT

(11) **EP 2 744 535 B1**
(45) Veröffentlichungstag und Bekanntmachung des Hinweises auf die Patenterteilung: **08.07.2015**
(21) Anmeldenummer: 12746102.8
(22) Anmeldetag: 14.08.2012
(51) Int. Cl.: A61M 1/16

(54) **DIALYSEVORRICHTUNG**
DIALYSIS DEVICE
DISPOSITIF DE DIALYSE

(30) Priorität: 18.08.2011 DE 102011081204
(43) Veröffentlichungstag der Anmeldung: 25.06.2014
(73) Patentinhaber: Dualis Medtech GmbH, 82229 Seefeld (DE)
(72) Erfinder: SCHILLER, Wolfgang, 53125 Bonn (DE); SCHMID, Thomas, 86938 Schondorf (DE); NGUYEN, Hiep Dr, Jamaica Plain MA 02130 (US)
(74) Vertreter: Von Kreisler Selting Werner - Partnerschaft von Patentanwälten und Rechtsanwälten mbB
(86) Internationale Anmeldenummer: PCT/EP2012/065886
(87) Internationale Veröffentlichungsnummer: WO 2013/024091

(56) Entgegenhaltungen:
- DE-A1-102008 017 448
- US-A- 3 974 854
- US-A- 4 524 466
- US-A- 5 397 354

## Beschreibung

### Hintergrund der Erfindung

Weltweit sind mehr als 1,5 Millionen Patienten nierenkrank und benötigen eine Nierenersatztherapie. Diese Zahl wird sich weiter mit einer jährlichen Rate von 6% erhöhen. Innovative Technologien und die Individualisierung des Behandlungsverfahrens können die Lebensqualität nierenkranker Patienten entscheidend verbessern. Ein besonderes Augenmerk gilt der Anpassung der Behandlung an die individuellen physiologischen Rahmenbedingungen der Patienten.

Die Dialyse kann aktuell die Funktion der Nieren nur unvollständig ersetzen. Es wird von verschiedenen Seiten daran gearbeitet die Entgiftung durch die Dialyse zu verbessern. Beim heutigen Stand der Technik stellt die Entgiftung durch dreimal wöchentliche Dialyse von je 4-5 Stunden eine sehr hohe körperliche und zeitliche Belastung für den Patienten dar. Durch die kurzen und intensiven Dialysezeiten wird das Zellgewebe des Patienten stark belastet (Plasmolyse), dies kann den gesundheitlichen Zustand des Patienten zusätzlich verschlechtern (Multimorbidität). Über die Jahrzehnte der Dialyse sammeln sich Giftstoffe an, so dass eine Reihe medizinischer Probleme auftreten können, wie z.B. Herzschwäche, Nervenstörungen, Knochenschmerzen.

Das Prinzip der Dialyse kann auf zwei Arten durchgeführt werden. Die erste Methode beruht auf dem Prinzip des osmotischen Effektes. Hierbei wird eine geeignete Membran verwendet, welche eine passende Porengröße aufweist, um zwischen einem Dialysat und dem Blut Abfallstoffe zu entfernen. Dieses bereits vielfach eingesetzte Verfahren benötigt jedoch, um einen angemessenen Effekt in möglichst kurzer Zeit zu erreichen, sehr große Oberflächen. Dieses Problem wird mittels herkömmlicher Dialysegeräte etwa durch die Verwendung einer Vielzahl von Membranröhren mit kleinstem Durchmesser erreicht. Jedoch ist hier dennoch eine Dialysezeit von mehreren Stunden erforderlich, und dies mehrmals wöchentlich. Ein weiteres Problem am Stand der Technik ist das immer wieder erforderliche Einstechen mit Nadeln in die Venen bzw. Artieren, um den Anschluss des Gerätes zu ermöglichen.

US 5,397,354 beschreibt eine Vorrichtung zum Reinigen des Blutes eines Patienten mit einer Blutkammer und eine Dialysatkammer, die durch eine Membran voneinander getrennt sind.

US 4,524,466 beschreibt eine kontinuierlich rotierende reversible Pumpe für ein Kunstherz. Zum Pumpen von Blut wird hier ein Hydraulikfluid verwendet, das von einer Blutkammer über eine Membran getrennt ist und diese komprimiert.

Mit der Erfindung soll die Aufgabe gelöst werden eine einfache, ressourcenschonende und für den Patienten gut verträgliche künstliche Niere zu schaffen. Es soll ein System geschaffen werden, das der Patient ständig an bzw. in seinem Körper tragen kann, ohne wesentlich an Bewegungsfreiheit und Lebensqualität eingeschränkt zu sein.

### Zusammenfassung der Erfindung

Die Erfindung betrifft eine tragbare Vorrichtung zur Dialyse welche teilweise oder komplett implantierbar ausgeführt werden kann. Gemäß einer Ausgestaltung der Erfindung besteht die Vorrichtung aus einer Blutkammer und einer Dialysekammer welche über einen geeigneten Filter verbunden sind, einem Gehäuse, einer elektrohydraulischen Pumpvorrichtung, sowie einem Reservoir.

Mittels einer geeigneten Flüssigkeit und einer hydraulischen Pumpvorrichtung wird die Blutkammer komprimiert und Blutplasma mit Stoffwechselabbauprodukten dem Kreislauf entnommen. Mittels eines extrakorporalen oder auch implantierbaren Reservoirs wird das Dialysat vorgehalten und verdünnt, so dass durch die Membran und den Pumpvorgang auch Flüssigkeit dem Körper zugeführt werden kann.

### Detaillierte Beschreibung bespielhafter Ausgestaltungen

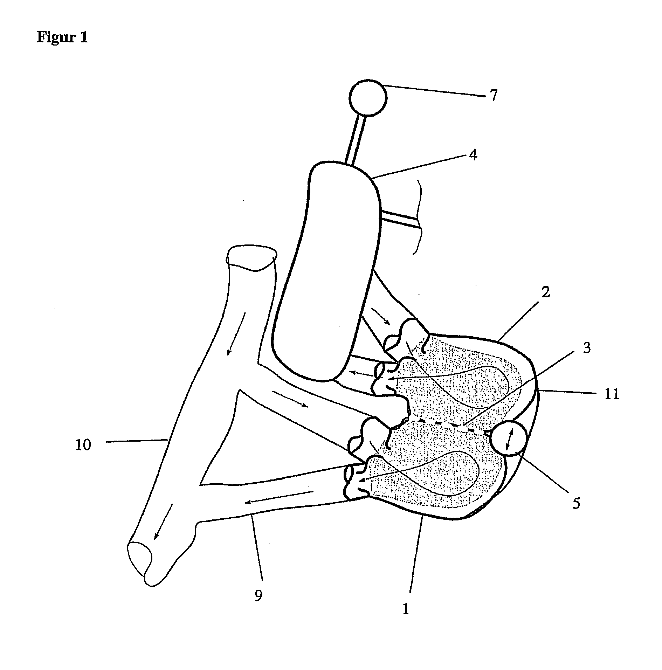

Die Erfindung betrifft ein tragbares Gerät zur Blutwäsche (Dialyse), welches teilweise oder ganz implantierbar sein kann (siehe Figur 1). Blut wird mit Hilfe einer Blutkammer 1 welche von Blut durchspült wird und einer Dialysatkammer 2, welche mittels einer Membran 3 verbunden sind, gereinigt. Dabei wird der osmotische Effekt zwischen der blutführenden Seite der Kammer und der Dialysatseite genutzt, um Abfallstoffe aus der Blutkammer durch die Membran zu ziehen. Weiterhin kann durch gezieltes Pumpen bzw. Saugen mittels einer Pumpe Blutplasma abgepresst bzw. Blutersatzflüssigkeit zurückgeführt werden. Die Porengröße der Membran ist günstigerweise in einer Größenordnung, die das Passieren von Stoffwechselabbauprodukten erlaubt, den Transport von Eiweiß weitgehend unterbindet. Die Blutkammer ist in einem festen oder teilweise festen Gehäuse 11 eingebaut, in welches das Hydraulikfluid gepumpt wird. Durch aktives Pumpen des Dialysates kann einer definierter Druck und Unterdruck auf die Membran 3 ausgeübt werden. Sie kann damit gezielt zusammendrückt und so das Blutvolumen in der Blutkammer ausgeworfen werden. Das Hydraulikfluid komprimiert die Kammern derart, dass ein Pumpeffekt entsteht und die Kammern günstig durchspült werden, so dass keine Stagnation in der Blutströmung auftritt, somit das Risiko von Thrombenbildung minimiert wird. Um Rückfluss zu vermeiden, werden die Kammern jeweils mit Ventilen ausgestattet. Durch die optimierte Durchströmung wird der Austauscheffekt der Membran zusätzlich verstärkt und die Effizienz der Blutreinigung erhöht. Durch die kontinuierliche Anwendung des Gerätes "24/7" (rund um die Uhr) kann eine dauerhafte und schonende Reinigung des Blutes erfolgen. Die Blutkammer wird günstigerweise an ein blutführendes Gefäß wie die Aorta 10 angeschlossen, kann aber auch etwa mit dem venösen System verbunden werden. Hierfür können handelsübliche Gefäßprothesen 9 verwendet werden. Für die Förderung des Hydraulikfluid kann eine geeignete Pumpe 5 eingesetzt werden.

Die Vorrichtung wird mit einem Reservoir 4 verbunden, welches das Dialysat vorhält. Die Vorrichtung und das Reservoir können implantierbar gestaltet sein. Sind beide Komponenten implantierbar, wird für das Wiederbefüllen des Reservoirs eine Perkutane Leitung bzw. ein handelsüblicher Port 7 vorgesehen. Durch den Einsatz einer separaten Kammer für das Dialysat, welche mit einem elektrohydraulischen Antrieb und Hydraulikfluid komprimiert wird, tritt eine effiziente Durchmischung des Dialysates und damit einer effiziente Verdünnung auf. Ferner wird durch den Einsatz elektrohydraulischer Antriebe ein kontrolliertes Befüllen und Entleeren der Blutkammer sicher gestellt, wodurch eine effiziente Durchspülung gewährleistet wird. Im Reservoir führt die Durchmischung zu einer Absenkung der Konzentration der Stoffwechselabbauprodukte, so dass bei einem erneuten Pumpvorgang nur in geringem Umfang diese Stoffe zurück in den Kreislauf gelangen. Dies ist möglich, solange der Sättigungsgrad einen Grenzwert nicht überschreitet. Dann muss die Flüssigkeit im Reservoir mittels geeigneter Methoden entweder gereinigt oder erneuert werden.

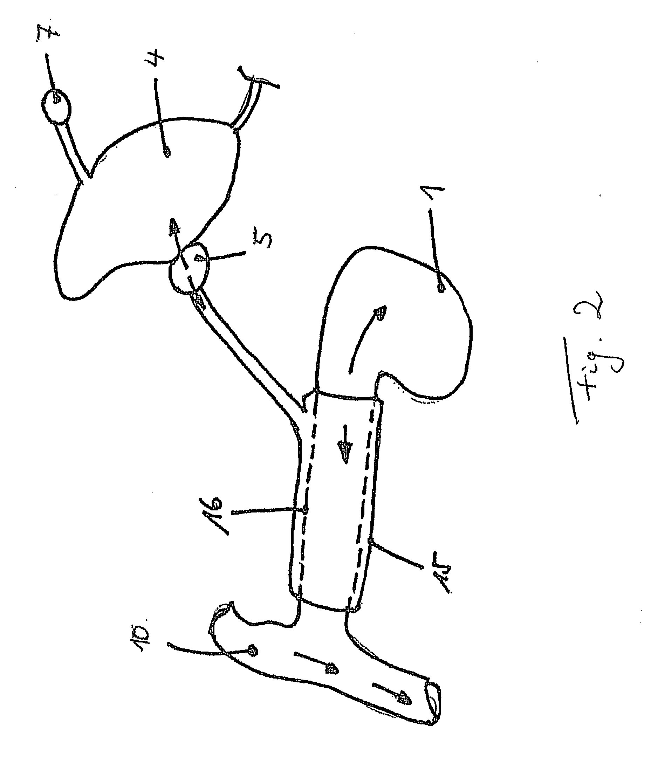

Mittels geeigneter Membrantechnologie kann diese Methode auch mit einer alternativen Anordnung durchgeführt werden. Eine beispielhafte Ausgestaltung ist in Fig. 2 gezeigt. Dabei wird ein vorzugsweise röhrenförmiger Zulauf 16 bzw. auch der Auslass der Pumpe als Membran ausgebildet und von einem größeren Rohrstück 15 umgeben, welches das Dialysat aufnimmt. Dabei kann entweder eine Membran verwendet werden, welche in beiden Richtungen die gleiche Durchlässigkeit aufweist, die Abfallprodukte müssen dann in geeigneter Weise aus dem Dialysat gefiltert bzw. gebunden werden, um nicht zurück in Blutbahn zu gelangen. Günstigerweise können die gelösten Abfallstoffe chemisch an größere Partikel gebunden werden, welche dann von der Membran zurückgehalten werden.

Alternativ kann die Membran oder deren Oberfläche mittels geeigneter Maßnahmen von Ihrer Ladung oder Oberflächenstruktur derart ausgebildet sein, dass Abfallstoffe abgestoßen werden und damit nicht zurück in die Blutbahn gelangen.

## Patentansprüche

1. Dialysevorrichtung, die zumindest teilweise in den Körper eines Patienten implantierbar ist, umfassend:
eine Blutkammer (1), durch die Blut des Patienten strömen kann;
eine Dialysekammer (2) zur Aufnahme eines Dialysats;
wobei beide Kammern (1,2) in einem Bereich miteinander verbunden sind, der als Membran (3) ausgebildet ist,
**gekennzeichnet durch**
eine hydraulische Pumpvorrichtung (5), um die beiden Kammern (1,2) mittels eines Hydraulikfluids wechselseitig zu komprimieren,
wobei beide Kammern (1,2) mit jeweils zwei Ventilen ausgestattet sind, um den Zu- und Abfluss von Blut bzw. Dialysat zu steuern.

2. Dialysevorrichtung nach Anspruch 1, umfassend eine Hydraulikpumpe (5) zum Erzeugen von Druck und Unterdruck, um mittels des Hydraulikfluids, insbesondere einem Dialysat, und der Membran (3) Blutplasma und Stoffwechselabbauprodukte aus dem Blutkreislauf des Patienten zu entnehmen und/oder dem Blutkreislauf Flüssigkeit zuzuführen.

3. Dialysevorrichtung nach Anspruch 1 oder 2, umfassend ein gegenüber dem Patienten externes oder in den Patienten implantierbares Reservoir (4) für eine geeignete Flüssigkeit zur Blutwäsche.

4. Dialysevorrichtung nach einem der vorhergehenden Ansprüche, wobei das Reservoir (4) einen Zugang (7) zum Befüllen des Reservoirs (4) aufweist.

5. Dialysevorrichtung nach einem der vorhergehenden Ansprüche, umfassend je einen Einlass und einen Auslass bei Blut- und Dialysekammer (1,2), die jeweils ein Ventil aufweisen.

6. Dialysevorrichtung nach einem der vorhergehenden Ansprüche, wobei die Dialysevorrichtung ein implantierbares Reservoir (4) umfasst, welches mittels einer perkutanen Leitung oder eines Ports (7) befüllbar ist.

7. Dialysevorrichtung nach einem der vorhergehenden Ansprüche, umfassend eine zumindest teilweise flexible Blutkammer (1), die im Wesentlichen rundformig oder ventrikelförmig ausgebildet ist.

8. Dialysevorrichtung nach einem der vorhergehenden Ansprüche, wobei die Dialysevorrichtung ausgebildet ist zur Abgabe von verbrauchtem Dialysat an den Harnleiter des Patienten.

## Claims

1. A dialysis device adapted to be implanted at least partially into the body of a patient, comprising:
a blood chamber (1), through which the patient's blood can flow;
a dialysis chamber (2) for receiving a dialysate;
wherein both chambers (1, 2) are connected to each other in an area designed as a membrane (3),
**characterized by**
a hydraulic pump device (5) for compressing the two chambers (1, 2) alternately by means of a hydraulic fluid,
wherein both chambers (1, 2) are each equipped with two valves for controlling the inflow and outflow of blood and dialysate, respectively.

2. The dialysis device of claim 1, comprising a hydraulic pump (5) for the generation of pressure and vacuum, for the purpose of using the hydraulic fluid, specifically a dialysate, and the membrane (3) to remove blood plasma and metabolic waste products from the blood circulation of a patient and/or to supply liquid into the blood circulation.

3. The dialysis device of claim 1 or 2, comprising a reservoir (4) for a liquid suitable for washing blood, the reservoir either being external with regard to the patient or being implantable into the patient.

4. The dialysis device of one of the preceding claims, wherein the reservoir (4) comprises a port (7) for the filling of the reservoir (4).

5. The dialysis device of one of the preceding claims, comprising an inlet and an outlet at the blood chamber and the dialysate chamber (1, 2), respectively, each comprising a valve.

6. The dialysis device of one of the preceding claims, wherein the dialysis device comprises an implantable reservoir (4) adapted to be filled through a percutaneous line or a port (7).

7. The dialysis device of one of the preceding claims, comprising an at least partially flexible blood chamber (1) that is substantially round or ventricle-- shaped.

8. The dialysis device of one of the preceding claims, wherein the dialysis device is designed to discharge used dialysate to the ureter of the patient.

## Revendications

1. Dispositif de dialyse, pouvant être implanté au moins partiellement, dans le corps d'un patient et comprenant :
une chambre à sang (1), à travers laquelle peut s'écouler du sang du patient ;
une chambre de dialyse (2) destinée à recevoir un dialysat ;
les deux chambres (1, 2) étant reliées l'une à l'autre dans une zone conçue en tant que membrane (3),
**caractérisé par**
un dispositif de pompage hydraulique (5), pour comprimer alternativement les deux chambres (1, 2) au moyen d'un fluide hydraulique,
les deux chambres (1, 2) étant chacune pourvues de deux soupapes pour commander l'arrivée et l'écoulement de sang ou de dialysat.

2. Dispositif de dialyse selon la revendication 1, comprenant une pompe hydraulique (5) destinée à la génération de pression et de dépression, afin de prélever du plasma sanguin et des produits de désassimilation issus de la circulation sanguine du patient et/ou approvisionner la circulation sanguine en liquide au moyen du fluide hydraulique, en particulier d'un dialysat, et de la membrane (3).

3. Dispositif de dialyse selon la revendication 1 ou 2, comprenant un réservoir (4) externe au patient ou pouvant être implanté dans le patient, destiné à contenir un liquide approprié à l'hémodialyse.

4. Dispositif de dialyse selon l'une des revendications précédentes, le réservoir (4) présentant un accès (7) pour le remplissage du réservoir (4).

5. Dispositif de dialyse selon l'une des revendications précédentes, (1, 2) comprenant au niveau de chacune des chambres à sang et de dialyse une entrée et une sortie présentant chacune une soupape.

6. Dispositif de dialyse selon l'une des revendications précédentes, le dispositif de dialyse comprenant un réservoir implantable (4) lequel peut être rempli au moyen d'un tube percutané ou d'un port (7).

7. Dispositif de dialyse selon l'une des revendications précédentes, comprenant une chambre à sang (1) au moins partiellement flexible qui présente une forme essentiellement arrondie ou ventriculaire.

8. Dispositif de dialyse selon l'une des revendications précédentes, le dispositif de dialyse étant conçu pour la distribution du dialysat usagé à l'uretère du patient.
